# EUROPEAN PATENT APPLICATION

(11) **EP 1 679 321 A1**
(43) Date of publication of application: **12.07.2006**
(21) Application number: 04799428.0
(22) Date of filing: 01.11.2004
(51) Int. Cl.: C08B 1/00, C07G 1/00, C07H 1/08, C13K 1/02

(54) **METHOD AND APPARATUS FOR MANUFACTURING LIGNOPHENOL DERIVATIVE**

(30) Priority: 31.10.2003 JP 2003371711
(71) Applicant: Functional Wood Material Research Association, Tokyo 1040032 (JP)
(72) Inventor: HAYASHI, Hideaki, c/o Ebara Corporation,, Tokyo 1448510 (JP); KAMIYA, Ichiro, c/o Ebara Corporation,, Tokyo 1448510 (JP); KONDO, Kazuhiro, c/o Ebara Corporation,, Tokyo 1448510 (JP)
(74) Representative: Wagner, Karl H.
(86) International application number: PCT/JP2004/016222
(87) International publication number: WO 2005/042586

(57) **Abstract**

It is an object to provide a method in which a lignocellulosic material is treated with a phenol derivative and an acid, whereby a lignophenol derivative can be produced and recovered efficiently, and moreover sugar from an acid/sugar mixture obtained at the same time can be recovered and used easily.

One form of the present invention relates to a method of preparing a lignophenol derivative and an acid/sugar mixture, comprising subjecting a reaction mixture of a lignocellulosic material, a phenol derivative and an acid to solid-liquid separation so as to separate into a solid-phase lignophenol derivative and a liquid-phase acid/sugar mixture, and then subjecting the separated lignophenol derivative to deacidification/washing.

## Description

### TECHNICAL FIELD

The present invention relates to a method and apparatus for efficiently separating and recovering a lignophenol derivative and sugar from a lignocellulosic material. By making use of the aromatic ring-containing structure of the lignophenol derivative obtained through the present invention, the lignophenol derivative can be used as a macromolecular material as a substitute for a petrochemical one.

### BACKGROUND ART

The use of fossil resources such as petroleum has become indispensable in modern society, but regeneration of fossil resources is impossible, and so it is feared that these resources will be exhausted in the near future. Interest in biomass resources as one type of resources for replacing fossil resources is thus increasing. Of biomass resources, ligneous biomass resources are receiving attention due to being enormously abundant on Earth, production being possible in a short time period, and sustained supply being possible through appropriate maintenance. Moreover, such ligneous biomass resources are also receiving more and more attention due to decomposing in the natural world after use as resources so as to be regenerated as new biomass resources. However, regarding the use of ligneous biomass resources (lignocellulosic material), hitherto the principal methods of use have been ones in which the carbohydrate (cellulose) is separated and recovered as pulp, or the cellulose and hemicellulose are solubilized with an acid and then recovered as sugar; the lignin contained in the ligneous biomass resources (lignocellulosic material) has usually been handled as residue, not being used as a resource. In the method in which the cellulose is recovered as pulp, the lignocellulosic material is digested with an alkali and thus separated into cellulosic fibrous matter and lignin, but in this case the lignin is fragmented to an extent making use as a material difficult. On the other hand, with the method in which the cellulose and hemicellulose in the lignocellulosic material are solubilized with an acid, although there is considered to be less denaturation of the lignin component compared with in the pulp industry, the lignin decomposed through being attacked by the acid undergoes recondensation due to the high reactivity thereof, becoming matter that is unsuitable for use as a macromolecular material.

So that the lignin in the lignocellulosic material can be effectively used, it is necessary to first separate the lignocellulosic material into the constituent components thereof, i.e. lignin, and cellulose and hemicellulose. As a technique for doing this, a method has been proposed in which a phenol derivative is impregnated into the lignocellulosic material, and then an acid is added, and the lignocellulosic material is separated into a lignophenol derivative and carbohydrate (Japanese Patent Application Laid-open No. 2-233701; "Synthesis of Functional Lignophenol Derivative using a Natural Lignin Phenol Derivative-Concentrated Acid Two-Phase System Treatment Method", Funaoka et al., Journal of Thermosetting Plastics, Japan, Vol. 15, No. 2 (1994), p. 77-87 (in Japanese); "Derivation of Phenolic Lignin Material using a Phase Separation Reaction System and Functions of the Material", Funaoka et al., Journal of Thermosetting Plastics, Japan, Vol. 16, No. 3 (1995), p. 151-165 (in Japanese)). According to the proposed method, a phenol derivative such as cresol is impregnated into a lignocellulosic material such as wood powder and solvation is carried out (i.e. the cresol is impregnated into the wood powder to produce a state in which the cresol is fixed close to the lignin in the wood powder), and then an acid is added so as to dissolve the cellulose component. At this time, cations at highly reactive sites of the lignin produced through contact with the acid are attacked by the phenol derivative, whereby the phenol derivative is introduced. Moreover benzyl aryl ether linkages are cleaved, whereby the molecular weight of the lignin is reduced. As a result, a lignophenol derivative in which the molecular weight of the lignin is reduced and the phenol derivative is introduced into benzylic positions of the basic structural units is produced. Next, the reaction system (here, this refers to the whole of the reaction liquid after the addition of the acid) is diluted with an excess of water so as to stop the reaction with the acid, and then the insoluble matter is collected together by centrifugal separation, whereby the lignophenol derivative is separated off.

### DISCLOSURE OF THE INVENTION

### PROBLEMS TO BE SOLVED BY THE INVENTION

However, in the above method, after the acid treatment, the reaction system is diluted with an excess of water, for example an amount of water at least 10 times the amount of the lignocellulosic material, and hence recovering the lignophenol derivative is difficult.
Furthermore, in the above method, through the acid treatment, as well as the lignophenol derivative being produced, the cellulose and hemicellulose in the lignocellulosic material are solubilized through the acid, being recovered as the liquid phase (an acid/sugar solution) after the lignophenol derivative has been separated off; however, because the reaction system (here, this refers to the whole of the reaction liquid after the addition of the acid) is diluted with an excess of water, the sugar concentration in the acid/sugar solution is too low, and thus it has been difficult in practice to separate out, recover, and use the sugar.

It is an object of the present invention to solve the above problem. That is, it is an object of the present invention to provide a method in which a lignocellulosic material is treated with a phenol derivative and an acid, whereby a lignophenol derivative can be produced and recovered efficiently, and moreover sugar from an acid/sugar mixture obtained at the same time can be recovered and used easily.

### MEANS FOR SOLVING THE PROBLEMS

As means for attaining the above object, the present invention provides a method of preparing a lignophenol derivative and an acid/sugar solution, comprising subjecting a reaction mixture of a lignocellulosic material, a phenol derivative and an acid to solid-liquid separation so as to separate into a solid-phase lignophenol derivative and a liquid-phase acid/sugar solution, and then subjecting the separated lignophenol derivative to deacidification/washing.

### BRIEF DESCRIPTION OF THE DRAWINGS

FIG. 1 is a flowchart schematically showing overall a process for manufacturing an acid/sugar solution and a lignophenol derivative from a lignocellulosic material using the present invention; and
FIG. 2 is a flowchart showing details of a deacidification/washing step carried out on lignophenol derivative-containing solid matter according to a preferable form of the present invention.

### MODE FOR CARRYING OUT THE INVENTION

Following is a description of a process for treating a lignocellulosic material according to the present invention. In the following, a description is given of the constitution of the present invention, and also the steps overall in a treatment process that uses the technical idea of the present invention and various representative forms of this treatment process. Accordingly, the technical scope of the present invention is stipulated by the claims, and is not limited by the following description.

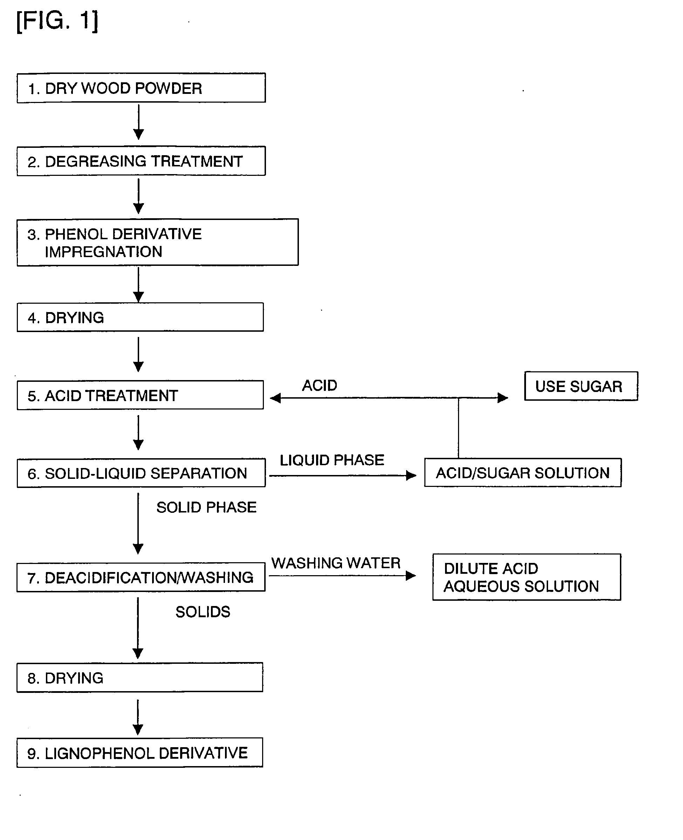

FIG. 1 is a flowchart schematically showing overall the process for separating an acid/sugar solution and a lignophenol derivative from a lignocellulosic material using the present invention. In the present invention, a "reaction mixture of the lignocellulosic material, a phenol derivative and an acid" can be prepared using, for example, a method publicly known in the technical field concerned. For example, a lignocellulosic material such as wood or a herbaceous material is first subjected to pre-treatment such as crushing and drying (1), and degreasing treatment is also carried out as required (2). Next, the phenol derivative is added to and thus impregnated into the lignocellulosic material (3).
Residual organic solvent is then dried off (4), and then the acid is added and agitation is carried out, whereby cell membranes in the lignocellulosic material are swollen and destroyed by the acid (5). As a result, the lignocellulosic material is decomposed into its component elements, i.e. cellulose, hemicellulose, and lignin. The decomposed lignin is reactively bonded to the phenol derivative that has already been added and thus impregnated in, and thus becomes hydrophobic solid matter containing the lignophenol derivative, and hence is protected from further decomposition by the acid. On the other hand, the molecular weight of the cellulose and hemicellulose is reduced by the acid, whereby solubilization of the cellulose and hemicellulose proceeds. In the present invention, the reaction liquid obtained through the above process is referred to as the "reaction mixture of the lignocellulosic material, the phenol derivative and the acid". In the present invention, the reaction mixture thus obtained is subjected to solid-liquid separation such as centrifugal separation as is without being diluted with water, and is thus separated into the hydrophobic solid matter containing the lignophenol derivative, and an acid/sugar solution containing the solubilized cellulose and hemicellulose (6). The hydrophobic solid matter containing the lignophenol derivative is subjected to deacidification/washing (7) to wash out and thus remove residual acid, and then the solid matter is recovered and subjected to a drying step (8), whereby the lignophenol derivative (9) is obtained.

Meanwhile, the acid/sugar solution obtained as the liquid phase through the solid-liquid separation (6) carried out on the reaction mixture after the acid treatment can be treated using a diffusion dialysis method, a simulated moving bed chromatography separation method, an alkanol solvent extraction method, or the like, whereby the sugar can be recovered.

Following is a detailed description of the various steps.

### Raw material pre-treatment step (1)

The lignocellulosic material, for example thinnings, wood residue of forestry land, sawmill waste, mill ends, herbaceous plants, rice husk, rice straw or the like is crushed. As ligneous raw material, cryptomeria or the like that is wood residue of forestry land or sawmill waste or the like can be suitably used. As herbaceous raw material, the crushed core of kenaf, which has attracted attention recently, or the like can be suitably used. After the crushing, sifting to a particle size of not more than 2 mm is preferably carried out, since this results in an effect of increasing the effectiveness of the subsequent impregnation with the phenol derivative and improving the reactivity. Moreover, it is preferable to carry out drying to a water content of approximately 15 to 20%, since then there is little sticking together of particles to form lumps during the sifting, and hence the yield of the raw material powder can be improved.

### Degreasing treatment (2)

Depending on the type of the lignocellulosic material, the lignocellulosic material may contain a large amount of resinous content or the like. It is preferable to remove the resinous content from the lignocellulosic material (i.e. carry out degreasing) before adding the phenol derivative, so that the resinous content will not inhibit the subsequent reaction process. As the degreasing method, the degreasing can be carried out, for example, by putting the lignocellulosic material and an organic solvent into an agitating tank, and thoroughly mixing and agitating. By carrying out such degreasing with an organic solvent, an effect of removing moisture from the lignocellulosic material is also obtained. Examples of organic solvents that can be used with this objective include acetone and hexane. The amount used of the organic solvent is preferably 1 to 10 times the amount of the lignocellulosic material. "X times the amount" stipulated here means X liters of the organic solvent per 1 kg of the wood powder, for example "10 times the amount" means that 10 L of the organic solvent is added per 1 kg of the wood powder. Moreover, it is preferable to carry out the degreasing thoroughly by agitating for 1 to 12 hours after the organic solvent has been added. The degreasing treatment is not an essential step, and need not be carried out, for example, in the case that there is not much resinous content in the lignocellulosic material being processed. In the case that the organic solvent used in the present degreasing step is different to an organic solvent used in the following phenol derivative impregnation step, it is preferable to dry the lignocellulosic material so as to remove the organic solvent used in the degreasing before carrying out the following phenol derivative impregnation. However, in the case that the same organic solvent is used in both steps, this drying/removal step may be omitted.

### Phenol derivative impregnation (3)

Next, a solution of the phenol derivative in an organic solvent is mixed with the lignocellulosic material and the mixture is thoroughly agitated, whereby the phenol derivative is impregnated into the lignocellulosic material. Phenol derivatives that can be used with this objective include p-cresol, m-cresol, o-cresol, and mixtures thereof, and also phenol. In this impregnation step, it is desirable to disperse the phenol derivative and impregnate the phenol derivative into the lignocellulosic material thoroughly, and to achieve this it is preferable to make the phenol derivative contact the lignocellulosic material in a state in which the phenol derivative has been mixed and dissolved in an organic solvent and thus thoroughly dispersed through the solvent. Moreover, to efficiently impregnate the phenol derivative into the lignocellulosic material, the solution of the phenol derivative in the organic solvent is preferably added in a proportion of 8 to 12 L per 1 kg of the lignocellulosic material after the degreasing treatment (here, this will be referred to as 8 to 12 times the amount of the lignocellulosic material), preferably approximately 10 times the amount of the lignocellulosic material, so that the impregnation step is carried out in a state in which the lignocellulosic material is thoroughly immersed in the phenol derivative solution. Moreover, the lignocellulosic material and the solution are preferably agitated for 1 to 24 hours at room temperature, for example 10 to 50°C, so that the impregnation proceeds sufficiently, with it being more preferable to maintain a temperature of approximately 30°C during the agitation. Examples of organic solvents that can be used for dissolving the phenol derivative include acetone and hexane; in the case of carrying out the degreasing step described above, the same organic solvent as that used in the degreasing step can be used. Examples of apparatuses that can be used for mixing and agitating the lignocellulosic material and the phenol derivative in the organic solvent include a conical ribbon mixer (RIBOCONE made by Okawara Mfg. Co., Ltd.). In the present step, the mixing can be carried out by adding the solution of the phenol derivative in the organic solvent into a mixing tank into which the lignocellulosic material has been put; in this case, it is preferable to reduce the pressure in the mixing tank into which the lignocellulosic material has been put before adding the phenol derivative, since then the penetrability of the phenol derivative into the gaps between the lignocellulosic material particles can be increased, and hence the penetrability of the phenol derivative into the lignocellulosic material cell walls can be increased. Furthermore, as the method of impregnating the phenol derivative into the lignocellulosic material, a pressurized injection method used, for example, for injecting preservatives into wood can be used. This is method in which the pressure in an injection tank into which the lignocellulosic material has been put is reduced, and then the phenol derivative is injected in under pressure. According to this method, the phenol derivative can be made to penetrate as far as the cell membranes of the lignocellulosic material. Note that "impregnation of the phenol derivative into the lignocellulosic material" in the present step does not necessarily mean that the phenol derivative is made to penetrate into the particles of the lignocellulosic material, but rather substantially the same effect can be obtained even if the phenol derivative is merely dispersed and attached very uniformly to the surfaces of the lignocellulosic material particles. This form is thus also included under "impregnation" in the present specification.

Moreover, the present inventors have discovered that in the step of impregnating the phenol derivative into the lignocellulosic material, instead of the method described above in which a phenol derivative solution is added in an amount approximately 10 times the amount of the lignocellulosic material so that the impregnation is carried out in a state in which the lignocellulosic material is thoroughly immersed in the solution, the phenol derivative can be dispersed and attached very uniformly to the surfaces of the lignocellulosic material particles and hence the desired effect can be obtained also through a method in which the phenol derivative solution is added to the lignocellulosic material in a small amount of approximately 1 to 5 times the amount of the lignocellulosic material while agitating the lignocellulosic material. The present invention also relates to such a method. That is, another form of the present invention relates to a method of impregnating the phenol derivative into the lignocellulosic material, in which a phenol derivative solution is added in an amount of 1 to 5 times, preferably approximately 1 times, relative to 1 kg of the crushed lignocellulosic material while agitating the lignocellulosic material. In this case, the amount added of the phenol derivative solution per 1 kg of the lignocellulosic material is more preferably 1 to 4 times, yet more preferably 1 to 2 times.

In this case, the impregnation of the phenol derivative into the lignocellulosic material is preferably carried out by spraying the phenol derivative solution onto the crushed lignocellulosic material while agitating the lignocellulosic material in an agitating apparatus capable of strongly agitating and mixing a powder. The agitating apparatus used in the present invention is an agitating apparatus having plough-shaped shovels and choppers; a stirrer to which these members are attached is rotated, whereby the crushed lignocellulosic material in the tank is subjected to a centrifugal dispersing action and a swirling action to form a state of three-dimensional flow; by spraying the phenol derivative solution onto the crushed lignocellulosic material in this state, a uniformly dispersed state can be realized even with a small amount of liquid. Furthermore, the drying off of the solvent after the impregnation step can also be carried out in the same strongly agitating apparatus, it being possible to greatly reduce the time required for the drying by using the same strongly agitating action as for the impregnation. An example of a strongly agitating apparatus that can be used with this objective is an MFK type mixer made by the German company Lödige.

By carrying out the impregnation of the phenol derivative into the lignocellulosic material using such a method, the amount used of the solvent can be greatly reduced, and moreover the impregnation can be made more uniform, and furthermore the time taken for the impregnation step can be greatly reduced. For example, with a method in which the impregnation is carried out by thoroughly immersing the lignocellulosic material in approximately 10 times the amount of the phenol derivative solution, it has taken approximately 2 to 3 days up to and including the drying step after the impregnation step, but with the above method, the impregnation and drying steps can be completed in only approximately 1 to 4 hours.

Note that in the case that the impregnation step is carried out by adding the phenol derivative solution to the crushed lignocellulosic material while agitating the lignocellulosic material as described above, in the case that the lignocellulosic material supplied in the impregnation step has had solvent remaining after the degreasing step described earlier removed by drying, or the solvent used in the degreasing step and the solvent used in the impregnation step are the same, the lignocellulosic material used may be obtained by draining off the solvent after the degreasing step (i.e. may having a small amount of the solvent remaining therein).

Furthermore, by carrying out impregnation of the phenol derivative into the lignocellulosic material by adding the phenol derivative solution in an amount of approximately 1 to 5 times relative to the crushed lignocellulosic material while strongly agitating the lignocellulosic material using a Lödige mixer or the like as described above, an effect is also produced whereby the concentration of the phenol derivative solution used in the impregnation can be reduced and hence the amount used of the phenol derivative can be reduced. To prepare the lignophenol derivative effectively, the amount of the phenol derivative impregnated into the lignocellulosic material must be approximately 0.1 to 0.5 kg of the phenol derivative per 1 kg of the lignocellulosic material. With a conventional method, to improve the effect of the impregnation of the phenol derivative into the lignocellulosic material, the impregnation has been carried out by thoroughly immersing the lignocellulosic material in approximately 10 times the amount of the phenol derivative solution. However, with that method, to reduce the heat expense of subsequent drying off of the solvent, a technique of draining off excess phenol derivative solution before the drying is adopted. In this case, the phenol derivative is removed together with the solvent, and hence it is usual to use the phenol derivative in a larger amount than the above, for example 0.3 to 1.5 kg per 1 kg of the wood powder, when carrying out the impregnation. However, according to the method in which the impregnation of the phenol derivative into the lignocellulosic material is carried out by adding the phenol derivative solution in an amount of approximately 1 to 5 times relative to the crushed lignocellulosic material while strongly agitating the lignocellulosic material using a Lödige mixer or the like as in the present invention, the amount of the phenol derivative used in the phenol derivative impregnation step can be made to be approximately 0.1 to 0.5 kg per 1 kg of the lignocellulosic material. As a result, the amount of the phenol derivative used can be greatly reduced, and moreover the time required for the impregnation and drying steps can be greatly reduced.

### Drying (4)

After the lignocellulosic material and the organic solvent solution having the phenol derivative dissolved therein have been thoroughly agitated so as carry out the impregnation, the pressure is reduced so that residual organic solvent is evaporated off at a low temperature, whereby the phenol derivative-impregnated lignocellulosic material is dried. In the case in particular of using acetone as the organic solvent for dissolving the phenol derivative, the acetone would dissolve the lignophenol derivative produced through the acid treatment in the next stage and thus inhibit the separation of the lignophenol derivative and the acid/sugar solution, and hence it is necessary to thoroughly remove residual acetone in the phenol derivative-impregnated lignocellulosic material before carrying out the acid treatment step.

### Acid treatment (5)

Next, the phenol derivative-impregnated lignocellulosic material is treated with an acid. As the acid used here, it is preferable to use concentrated sulfuric acid of concentration at least 65%, and to sustain the reactivity, it is more preferable to use concentrated sulfuric acid of concentration at least 72%. The amount of the acid added is preferably 1 to 10 times the amount, more preferably 3 to 5 times the amount, of the lignocellulosic material. "X times the amount" for the acid here means X liters of the acid per 1 kg of the lignocellulosic raw material before the impregnation of the phenol derivative (i.e. not including the weight of the impregnated phenol derivative), for example "10 times the amount" means that 10 L of the acid is added per 1 kg of the lignocellulosic raw material not including the weight of the impregnated phenol derivative. In the acid treatment step, it is preferable to add the acid after the phenol derivative-impregnated lignocellulosic material has been put into the reaction tank, since then a reaction time difference can be eliminated, and hence the acid treatment can be carried out uniformly; however, there is no limitation to this, but rather a method in which the phenol derivative-impregnated lignocellulosic material is mixed in after the acid has been put into the reaction tank is also possible. After the phenol derivative-impregnated lignocellulosic material and the acid have been mixed together, agitation must be carried out thoroughly and uniformly so as to make the reaction proceed uniformly; however, immediately after the mixing in of the acid, the phenol derivative-impregnated lignocellulosic material has a very high viscosity, and hence is not easily agitated. The present inventors have discovered that if a planetary agitation type kneader is used in the acid treatment step, then reliable mixing and agitation is possible even in the initial high-viscosity state, and hence the acid treatment can be carried out efficiently.

As art for hydrolyzing a lignocellulosic material with an acid, there have been a dilute acid method, a concentrated acid method, and so on from hitherto, but all of these have been used with an objective of solubilizing cellulose and hemicellulose and separating out the sugar, and have not been used for separating out and recovering the lignin. For example, with the dilute acid method, the lignocellulosic material is subjected to the acid treatment under high-temperature high-pressure conditions, but under such conditions, the lignin is sulfonated or carbonized, making effective use thereof difficult. In the present method, the reaction of decomposing the lignocellulosic material with the acid into the lignophenol derivative and the acid/sugar solution takes place under normal temperature and pressure. To uniformly maintain the reactivity while preventing carbonization or sulfonation of the lignophenol derivative produced, the acid treatment reaction in the present method is preferably carried out at a temperature of 20 to 40°C, preferably approximately 30°C. Moreover, to prevent denaturation of the lignophenol derivative by the acid, the reaction time for the acid treatment is preferably 10 minutes to 2 hours, more preferably 30 minutes to 1 hour.

As a control method for holding the acid treatment reaction temperature constant, for example the acid treatment reaction tank may have a warm water jacket through which warm water is passed provided on the outside of the reaction tank, and an apparatus for measuring the temperature of the reaction mixture in the reaction tank. When carrying out the acid treatment reaction, warm water of the preset reaction temperature is passed through the warm water jacket, so that the temperature of the whole of the reaction tank constituting the reaction environment is held at the desired acid treatment reaction temperature. Then after the raw material has been put into the reaction tank and the acid treatment reaction has begun, the temperature and flow rate of the warm water being passed through the warm water jacket are adjusted while monitoring the temperature of the reaction liquid using the temperature measuring apparatus provided in the reaction tank, whereby changes in the temperature of the reaction environment due to the heat of reaction can be absorbed. The present invention also relates to this acid treatment reaction control apparatus. That is, one form of the present invention relates to an acid treatment reaction apparatus for reacting an acid with a phenol derivative-impregnated lignocellulosic material so as to produce a lignophenol derivative and an acid/sugar solution, the apparatus comprising: a reaction tank that receives the phenol derivative-impregnated lignocellulosic material and the acid, and is for carrying out the reaction; a warm water jacket provided on the outside of the reaction tank; means for supplying and discharging warm water into and out of the warm water jacket; a temperature measuring apparatus for measuring the temperature of the contents of the reaction tank; and control means for adjusting the temperature and flow rate of the warm water supplied into the warm water jacket in accordance with the temperature of the contents measured by the temperature measuring apparatus.

Through this acid treatment step, cations at highly reactive sites of the lignin produced through contact with the acid are attacked by the phenol derivative, whereby the phenol derivative is introduced. Moreover benzyl aryl ether linkages are cleaved, whereby the molecular weight of the lignin is reduced. As a result, a lignophenol derivative in which the phenol derivative is introduced into benzylic positions of the basic structural units is produced. Moreover, at the same time, cellulose and hemicellulose in the lignocellulosic material are solubilized by the acid, and thus dissolve in the acidic solution. In the present invention, the mixture of the lignophenol derivative and the acid/sugar solution thus obtained is referred to as the "reaction mixture of the lignocellulosic material, the phenol derivative and the acid".

### Solid-liquid separation (6)

In one form of the present invention, the reaction mixture of the lignocellulosic material, the phenol derivative and the acid obtained as described above is subjected to a solid-liquid separation step so as to separate into a solid phase containing the lignophenol derivative, and a liquid phase of the acid/sugar solution having cellulose and hemicellulose dissolved therein. Centrifugal separation can be used in this solid-liquid separation step. As a centrifugal separator that can be used with this objective, a hole-less bottom discharge type centrifugal separator can be used. Using a hole-less bottom discharge type centrifugal separator is suitable, since then the sticky lignophenol derivative solid matter can be separated from the acid/sugar solution with no clogging. Here, it is preferable to carry out the centrifugal separation for 10 to 60 minutes. Through the centrifugal separation, the hydrophobic solid matter containing the lignophenol derivative, and the acid/sugar solution having the cellulose and hemicellulose dissolved therein are separated into two layers on the inside and outside respectively in the basket of the centrifugal separator due to the difference in density therebetween. Upon stopping the rotation of the centrifugal separator, the acid/sugar solution on the outside is discharged under its own weight from a discharge port provided in a lower portion of the apparatus. After the acid/sugar solution has been discharged, the lignophenol derivative-containing hydrophobic solid matter remaining in the basket is discharged from the discharge port in the lower portion of the apparatus using a scraper or the like.

Moreover, membrane separation using a filter or the like can also be used in this solid-liquid separation. In this case, after the acid treatment, the reaction mixture is introduced into a filtration tank in which a filter has been laid, and the lignophenol derivative-containing hydrophobic solid matter is separated by filtration from the acid/sugar solution having the cellulose and hemicellulose dissolved therein under the liquid's own weight or by using reduction of pressure or application of pressure. Here, the filtration tank preferably has a structure such as to enable liquid collection so that the filtration can be carried out after a suitable amount of the liquid has been collected. By using a filtration tank having such a structure, the thickness of the sticky lignophenol derivative-containing hydrophobic solid matter filter cake can be secured, and hence the ability to remove and recover the solid matter can be improved. Moreover, when carrying out the filtration, it is also possible to reduce the pressure so as to carry out the filtration and remove the liquid, and then apply pressure for a suitable time, whereby the removal of the liquid from the solid matter can be improved, and the ability to remove the filter cake can be improved. Furthermore, by using a flat plate-shaped filter cloth, the ability to remove the lignophenol derivative-containing solid matter after the liquid has been removed can be improved, and washing of the solid matter remaining on the surface of the filter cloth becomes easier. The filter cloth washing water can be used as the dispersing liquid in a subsequent step of dispersing the lignophenol derivative-containing solid matter in water after the liquid has been removed.

The lignophenol derivative-containing solid matter obtained through the above solid-liquid separation treatment is subjected to a deacidification/washing step and a drying step, described below, whereby the lignophenol derivative can be recovered. Meanwhile, regarding the acid/sugar solution containing solubilized cellulose and hemicellulose separated off and recovered as the liquid phase, the acid and the sugar can be separated and recovered using a method publicly known in the technical field concerned (e.g. a diffusion dialysis method, a simulated moving bed chromatography separation method, an alkanol solvent extraction method, etc.) or the like. The sugar recovered through the separation can, for example, be used as a raw material for biodegradable plastic manufacture using, for example, lactic acid fermentation, and the acid can be reused in the previous acid treatment step (5). According to the present invention, after the acid treatment, the reaction mixture is not diluted with a large amount of water as in the prior art, but rather is subjected to the solid-liquid separation to separate and recover the solid phase and the liquid phase as is without being diluted. As a result, a high-concentration acid/sugar solution is obtained, and hence the subsequent treatment to separate and recover the sugar and the acid can be carried out efficiently. Moreover, because the acid recovered through carrying out the separation on the acid/sugar solution is not diluted with water, refining such as concentration can be carried out with little heat expense, with the concentrated acid obtained through the refining being reused in the previous acid treatment.

### Deacidification/washing (7)

Acid, dissolved carbohydrate, and unreacted matter remain in the lignophenol derivative-containing solid matter obtained through the above solid-liquid separation treatment (6), and hence this residual matter must be removed by washing (deacidification/washing treatment). As has been done conventionally, this can be carried out by repeating a suitable number of times an operation of dispersing the lignophenol derivative-containing solid matter in at least 10 times the amount of water and agitating so as to cause the residual acid and so to move to the aqueous side, then leaving to stand so that the solid matter settles naturally, and then removing the supernatant. By dispersing the solid matter in water, the concentration of the acid is diluted and hence the reaction with the acid is stopped at the same time.

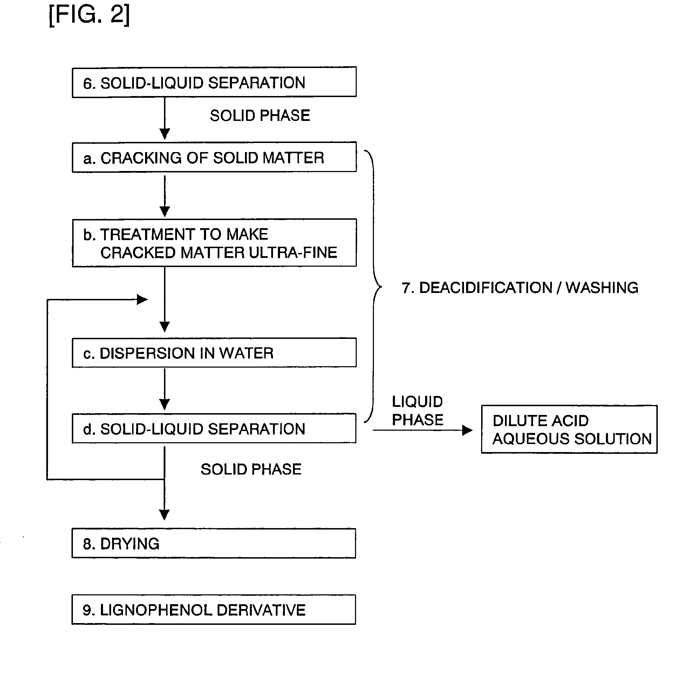

However, with the above method, because the lignophenol derivative-containing solid matter is solid and sticky, dispersing the solid matter in water is not easy, and moreover the solid matter takes a long time to settle after the agitation, and hence this method has sometimes required several days to several tens of days. Consequently, in another form of the present invention, there is provided a technique for carrying out the step of deacidifying/washing the lignophenol derivative-containing solid matter efficiently in a short time. As this technique, the present invention provides a method of recovering a lignophenol derivative, comprising adding water to the lignophenol derivative obtained as the solid phase through the solid-liquid separation carried out on the reaction mixture of the lignocellulosic material, the phenol derivative and the acid, and crushing so as to obtain a fine slurry, next dispersing the fine slurry obtained in water, and then recovering the solid matter. The method of deacidifying/washing the lignophenol derivative-containing solid matter according to this form of the present invention is shown schematically as a flowchart in FIG. 2.

In the method shown in FIG. 2, the lignophenol derivative-containing solid matter obtained through the solid-liquid separation treatment (6) is first crushed to obtain a fine slurry (a). The crushing can be carried out, for example, by putting the solid matter into an apparatus having impellers that rotate at high speed in a lower portion of a tank (e.g. a "cutter mixer"), and adding a suitable amount of water and agitating. Here, the amount of water added is preferably 1 to 5 times the amount of the solid matter. "X times the amount" stipulated here means X liters of water per 1 kg of the solid matter, for example "5 times the amount" means that 5 L of water is added per 1 kg of the solid matter. Through adding water and dispersing in this way, the concentration of the acid is diluted and hence the reaction with the acid is stopped at the same time. An example of a crushing apparatus that can be used with the above objective is a HIGHSPEEDER made by Pacific Machinery & Engineering Co., Ltd.

The fine slurry of the lignophenol derivative-containing solid matter obtained through the crushing is preferably further made ultra-fine (emulsified) using an apparatus such as a line mixer that makes the solid matter fine through shear, whereby the efficiency of the deacidification can be further improved (b). An example of an apparatus for making the solid matter fine that can be used with this objective is a Fine Flow Mill made by Pacific Machinery & Engineering Co., Ltd.

Next, a suitable amount of water is added to the ultra-fine slurry of the lignophenol derivative-containing solid matter obtained through the crushing and making ultra-fine and agitation is carried out thoroughly, whereby acid, dissolved carbohydrate, and unreacted matter remaining in the lignophenol derivative-containing solid matter are caused to move to the aqueous side and are thus diluted (c. dispersion in water). The amount of water added here is preferably 5 to 10 times (weight ratio) the amount of the lignophenol derivative-containing solid matter obtained through the solid-liquid separation.

After acid, dissolved carbohydrate, and unreacted matter remaining in the solid matter have been caused to move to the aqueous side through the dispersion in water, the aqueous phase is removed, and then the solid matter is again dispersed in water; by repeating this process a suitable number of times, the lignophenol derivative-containing solid matter can be deacidified/washed. Note that lignophenol derivative-containing solid matter remains in the tanks of the apparatuses in the previous steps of crushing and making ultra-fine. It is thus possible to use the discharged liquid obtained through washing these apparatus tanks with water as the dispersing liquid in the first step of dispersing in water, whereby the lignophenol derivative-containing solid matter recovery rate can be improved. Note, however, that because this discharged liquid also contains acid remaining in the tanks of the apparatuses for the crushing and making ultra-fine, from the perspective of the efficiency of the deacidification, it is undesirable to use the discharged liquid as the dispersing liquid in the second and subsequent steps of dispersing in water.

As the method of removing the liquid after the dispersion in water, for example a method can be adopted in which the aqueous slurry obtained by thoroughly agitating the dispersion is left to stand for a suitable time so that the solid matter settles, and then the supernatant is discharged. After the supernatant has been discharged, fresh water is added and dispersion in water is carried out again; this process can be repeated a suitable number of times. This method is a simple method that can be implemented with equipment comprising only a dispersion tank and a stirrer, but there is a problem that when the supernatant is discharged some of the lignophenol derivative-containing solid matter is discharged therewith, and hence the lignophenol derivative-containing solid matter recovery rate drops.

Consequently, in a preferable form of the present invention, the aqueous slurry obtained by thoroughly agitating the dispersion is separated into the solid matter and a liquid component using a solid-liquid separation apparatus (d), and then the solid matter is again subjected to dispersion in water, with this process being repeated a suitable number of times as required, whereby the efficiency of removing the liquid from the lignophenol derivative-containing solid matter, i.e. the efficiency of the deacidification, can be improved, and moreover loss of solid matter can be prevented. Because the lignophenol derivative-containing solid matter is sticky, a good liquid removal effect cannot be obtained if a generally used solid-liquid separation apparatus such as a decanter is used. Moreover, with a centrifugal dehydrator, the liquid can be removed from the lignophenol derivative-containing solid matter, but because the filter cloth laid in the basket is three-dimensionally sewn, taking out the solid matter after the liquid has been removed is difficult, with much solid matter remaining on the surface of the filter cloth. Moreover, when washing the filter cloth, it is often difficult to wash only the surface on which solid matter is attached. Consequently, in a preferable form of the present invention, it is preferable to subject the aqueous slurry of the lignophenol derivative-containing solid matter to solid-liquid separation using a filtration apparatus. As a result, the water removal can be carried out without compacting of the sticky lignophenol derivative-containing solid matter. As a filtration apparatus that can be used with this objective, a vacuum filtration apparatus is preferable, with a vacuum filtration apparatus having a structure enabling liquid collection so that the vacuum filtration can be carried out after a suitable amount of the liquid has been stored being particularly preferable. By using a filtration apparatus having such a structure, the cake thickness of the sticky lignophenol derivative-containing hydrophobic solid matter can be secured, and hence the ability to remove and recover the solid matter from filter surface can be improved. Moreover, when carrying out the filtration, after carrying out the filtration and liquid removal using a vacuum, it is possible to apply pressure for a suitable time, so as to further promote the removal of the liquid from the solid matter, and improve the ability to remove the cake. Moreover, when carrying out the filtration, it is preferable to use a flat plate-shaped filter cloth, since then the ability to remove the solid matter after the liquid has been removed can be improved, and washing of the solid matter remaining on the surface of the filter cloth becomes easier. The filter cloth washing water can be used as the dispersing liquid when repeatedly washing the solid matter.

Moreover, in the deacidification/washing step (7), by using an apparatus having an agitating mechanism in a tank and a filter in the bottom, the dispersion in water and the solid-liquid separation can be carried out in the same apparatus tank. In this case, the slurry obtained through the crushing and making fine is put into the tank, then a suitable amount of fresh water is added and agitation is carried out thoroughly, and then the lignophenol derivative-containing solid matter, and an aqueous phase into which the acid component and so on has moved and been diluted can be separated through filtration by the filter in the bottom of the tank. In this case, the amount of water added is preferably made to be 5 to 10 times (weight ratio) the amount of the lignophenol derivative-containing solid matter obtained through the solid-liquid separation.

As described above, it is preferable to repeat the deacidification/washing until the acid concentration in the supernatant from the dispersing water, or the aqueous phase (filtrate) obtained through the solid-liquid separation after the dispersion in water becomes low. Specifically, it is preferable to repeat the deacidification/washing process until the pH of the supernatant or the filtrate becomes at least 5. According to prototype tests carried out by the present inventors using the constitution of the present invention described above, it was possible to make the pH of the filtrate be at least 5 by repeating the dispersion in water and solid-liquid separation 4 to 8 times.

### Drying (8)

After the deacidification/washing of the lignophenol derivative-containing solid matter has been completed, the solid matter is recovered and dried. Utilizing the property that the lignophenol derivative will dissolve in acetone, the recovered lignophenol derivative-containing solid matter is mixed with acetone, so as to extract only the lignophenol derivative. The extract can be used by being impregnated into a material such as wood, but in this case, if there is residual moisture present when mixing with the acetone, then residual sugar in the lignophenol derivative-containing solid matter will dissolve into the acetone via the moisture, making it difficult to produce a pure lignophenol derivative acetone solution. It is thus preferable to dry the lignophenol derivative-containing solid matter as far as a water content of approximately not more than 5%.

Conventionally, natural drying has been predominantly used for drying such lignophenol derivative-containing solid matter, but one week to several months has been required to carry out the drying sufficiently. In the present invention, to reduce the time required for the drying and thus improve the production efficiency, it is preferable to subject the solid matter first to rough drying to a water content of not more than 50% through drying in a natural air current or drying by blasting with warm air, and then to high-level drying to a water content of not more than 10%. The temperature of the lignophenol derivative during the rough drying is preferably made to be not more than 60°C, and to improve the quality of the lignophenol derivative is more preferably made to be not more than 40°C. In the rough drying, it is preferable to spread the solid matter over a water-absorbent substance, and carry out drying in a natural air current or a warm air blast. The high-level drying can be carried out, for example, by using a vacuum microwave drier, putting the lignophenol derivative-containing solid matter that has been subjected to the rough drying to a water content of not more than 50% into a drying chamber of the drier, reducing the pressure in the drying chamber so as to make the evaporating temperature of water not more than 40°C, and then irradiating the solid matter in the drying chamber with microwaves so as to heat and thus evaporate off the contained moisture. Moreover, by using the above in combination with irradiation of far infrared radiation in the drying chamber, the drying efficiency can be further improved.

The lignophenol derivative obtained through the above process can be used in any of various fields as a petroleum-substitute macromolecular material.

The present invention further relates to an apparatus for implementing a method as described above. Specifically, another form of the present invention relates to an apparatus for recovering a lignophenol derivative, comprising: a crushing apparatus that receives solid matter obtained through solid-liquid separation carried out on a reaction mixture of a lignocellulosic material, a phenol derivative and an acid, and is for crushing the solid matter; an agitating tank for adding water to the crushed solid matter and agitating; and a solid-liquid separation apparatus that receives an aqueous slurry recovered from the agitating tank, and is for carrying out solid-liquid separation. Moreover, the present invention also relates to an apparatus for recovering a lignophenol derivative, comprising: a first solid-liquid separation apparatus for carrying out solid-liquid separation on a reaction mixture of a lignocellulosic material, a phenol derivative and an acid; a crushing apparatus that receives solid matter recovered through the first solid-liquid separation, and is for crushing the solid matter; an agitating tank for adding water to the crushed solid matter and agitating; and a second solid-liquid separation apparatus that receives an aqueous slurry recovered from the agitating tank, and is for carrying out solid-liquid separation. Furthermore, the present invention also relates to an apparatus for recovering a lignophenol derivative, comprising: an acid treatment tank that receives a phenol derivative-impregnated lignocellulosic material, and is for adding an acid to bring about reaction; a first solid-liquid separation apparatus that receives a reaction mixture of the lignocellulosic material, the phenol derivative and the acid recovered from the acid treatment tank, and is for carrying out solid-liquid separation; a crushing apparatus that receives solid matter recovered through the first solid-liquid separation, and is for crushing the solid matter; an agitating tank for adding water to the crushed solid matter and agitating; and a second solid-liquid separation apparatus that receives an aqueous slurry recovered from the agitating tank, and is for carrying out solid-liquid separation.

### Examples

The present invention will now be described in more detail through the following examples. However, the present invention is not limited to the following description.

### Example 1

A cryptomeria wood powder obtained by crushing cryptomeria chips, then drying, and then sifting to 0.2 to 2 mm was used as a raw material. 150 kg of the cryptomeria wood powder was put into a conical ribbon mixer (RIBOCONE made by Okawara Mfg. Co., Ltd.), 1500 L of acetone was added, and agitation was carried out for approximately 6 hours, and then the mixture was left to stand for 24 hours, thus carrying out first degreasing treatment. 1000 L of acetone was then discharged, the same amount of acetone (1000 L) as the discharged amount was re-added, and agitation was carried out for approximately 4 hours, thus carrying out second degreasing treatment. 1000 L of acetone was then discharged, and then a mixture of 75 kg of p-cresol and 800 L of acetone was added, and agitation was carried out thoroughly for 4 hours, thus impregnating the p-cresol into the cryptomeria wood powder. After leaving to stand for 24 hours, the pressure in the tank was reduced, thus thoroughly drying off residual acetone (over approximately 1 day). The above degreasing and p-cresol impregnation were carried out at room temperature (15°C). 225 kg of p-cresol-impregnated cryptomeria wood powder was obtained.

22.5 kg of the p-cresol-impregnated cryptomeria wood powder was put into an agitating reaction tank, and 72% sulfuric acid was added in an amount of 75 L, i.e. 5 times the amount relative to the cryptomeria wood powder, thus carrying out acid treatment. The agitating reaction tank and the added sulfuric acid used in the acid treatment were warmed to a temperature of 30°C in advance and held at this temperature. The mixture was agitated thoroughly for 1 hour in the reaction tank so as to cause the reaction to proceed, and then the mixture was subjected to solid-liquid separation treatment using a hole-less bottom discharge type centrifugal separator. After a separating time of approximately 10 minutes had elapsed, in the centrifugal separator separation had taken place into a solid-phase lignophenol derivative on the inside (i.e. at the center) and a liquid-phase sulfuric acid/sugar solution on the outside (i.e. at the periphery). Upon stopping the rotation of the centrifugal separator, the liquid-phase sulfuric acid/sugar solution was discharged from a discharge port provided in a lower portion of the centrifugal separator. The solid-phase lignophenol derivative remained in the basket of the centrifugal separator in a belt shape, and hence was scraped off using a scraper installed in the centrifugal separator, and thus made to drop down into the discharging section.

35 kg of the separated lignophenol derivative-containing solid matter was transferred into a crushing apparatus (HIGHSPEEDER made by Pacific Machinery & Engineering Co., Ltd.), and approximately 70 L of water was added and crushing treatment was carried out, whereby the solid matter was dispersed in the water. The dispersion was passed through a line mixer (Fine Flow Mill made by Pacific Machinery & Engineering Co., Ltd.) so as to make the solid matter ultra-fine with a particle size of not more than 0.1 mm, and then the dispersion was agitated while putting in water so as to make the final amount of the dispersion 200 L. The dispersion was then repeatedly subjected to filtration using a vacuum filtration apparatus, whereby the lignophenol derivative from which the sulfuric acid and sugar components had been removed was recovered as the solid matter.

Conventionally, when carrying out such washing of the lignophenol derivative with water, dispersion in water is carried out after carrying out only crushing, then agitation is carried out for approximately 2 hours, then the mixture is left to stand for 24 hours, and then the next day, in a state in which the lignophenol derivative-containing solid matter has settled naturally, the supernatant is discharged, and then fresh water is re-added and agitation is carried out; these steps are repeated, whereupon generally it has taken approximately 10 days until lignophenol derivative from which the sulfuric acid has been sufficiently removed (it is judged that the sulfuric acid has been removed once the pH of the dispersion has become at least 5) is recovered, and moreover solid matter remains in the supernatant, and hence the recovery rate has not been sufficiently high. In contrast with this, in the method according to the present invention described above, dispersion in water is carried out after carrying out crushing and making the solid matter ultra-fine, and then the dispersion is filtered using a vacuum filtration apparatus and the liquid is removed, whereby a sulfuric acid-containing liquid component can be removed efficiently, and moreover the loss of solid matter can be minimized. Moreover, by repeating the treatment of again dispersing the obtained lignophenol derivative in water and carrying out vacuum filtration, residual sulfuric acid in the lignophenol derivative can be completely removed efficiently. In the present invention, upon subjecting to vacuum filtration the solution obtained by crushing and making ultra-fine and then dispersing in 200 L of water the 35 kg of belt-shaped lignophenol derivative-containing solid matter separated from the reaction liquid obtained by carrying out the acid treatment on the 22.5 kg of p-cresol-impregnated cryptomeria wood powder, and then repeating the steps of again dispersing the solid matter obtained in 200 L of water and carrying out vacuum filtration 5 to 7 times, the pH of the filtrate became at least 5 and hence it was determined that the sulfuric acid had been sufficiently removed; this process of deacidifying/washing the lignophenol derivative, which has required approximately 10 days conventionally, could be completed in approximately 1 day. Moreover, the amount of the lignophenol derivative ultimately obtained was 6.5 kg (in terms of dry matter), and hence a yield double that conventionally obtained could be obtained.

### Example 2

A cryptomeria wood powder obtained by crushing cryptomeria chips, then drying, and then sifting to 0.2 to 2 mm was used as a raw material. 150 kg of the cryptomeria wood powder was put into a conical ribbon mixer (RIBOCONE made by Okawara Mfg. Co., Ltd.), 1500 L of acetone was added, and agitation was carried out for approximately 6 hours, and then the mixture was left to stand for 24 hours, thus carrying out first degreasing treatment. 1000 L of acetone was then discharged, the same amount of acetone (1000 L) as the discharged amount was re-added, and agitation was carried out for approximately 4 hours, thus carrying out second degreasing treatment. 1000 L of acetone was then discharged, and then a mixture of 215 kg of p-cresol (i.e. 1.4 kg per 1 kg of the cryptomeria wood powder) and 780 L of acetone was added, and agitation was carried out thoroughly for 4 hours, thus impregnating the p-cresol into the cryptomeria wood powder. After leaving to stand for 24 hours, 1000 L of excess p-cresol acetone solution in the tank was discharged, whereupon a prescribed amount of p-cresol remained in the cryptomeria wood powder. After the discharging of the excess liquid had been completed, the pressure in the tank was reduced, thus thoroughly drying off residual acetone (over approximately 1 day). The above degreasing and p-cresol impregnation were carried out at room temperature (15°C). 220 kg of p-cresol-impregnated cryptomeria wood powder was obtained.

22 kg of the p-cresol-impregnated cryptomeria wood powder was put into an agitating reaction tank, and 72% sulfuric acid was added in an amount of 72 L, i.e. 5 times the amount relative to the cryptomeria wood powder, thus carrying out acid treatment. The agitating reaction tank and the added sulfuric acid used in the acid treatment were warmed to a temperature of 30°C in advance and held at this temperature. The mixture was agitated thoroughly for 1 hour in the reaction tank so as to cause the reaction to proceed, and then the mixture was subjected to solid-liquid separation treatment using a hole-less bottom discharge type centrifugal separator. After a separating time of approximately 10 minutes had elapsed, in the centrifugal separator separation had taken place into a solid-phase lignophenol derivative on the inside (i.e. at the center) and a liquid-phase sulfuric acid/sugar solution on the outside (i.e. at the periphery). Upon stopping the rotation of the centrifugal separator, the liquid-phase sulfuric acid/sugar solution was discharged from a discharge port provided in a lower portion of the centrifugal separator. The solid-phase lignophenol derivative remained in the basket of the centrifugal separator in a belt shape, and hence was scraped off using a scraper installed in the centrifugal separator, and thus made to drop down into the discharging section.

35 kg of the separated lignophenol derivative-containing solid matter was transferred into a crushing apparatus (HIGHSPEEDER made by Pacific Machinery & Engineering Co., Ltd.), and approximately 70 L of water was added and crushing treatment was carried out, whereby the solid matter was dispersed in the water. The dispersion was passed through a line mixer (Fine Flow Mill made by Pacific Machinery & Engineering Co., Ltd.) so as to make the solid matter ultra-fine with a particle size of not more than 0.1 mm, and then the dispersion was agitated while putting in water so as to make the final amount of the dispersion 200 L. The dispersion was then repeatedly subjected to filtration using a vacuum filtration apparatus, whereby the lignophenol derivative from which the sulfuric acid and sugar components had been removed was recovered as the solid matter.

Conventionally, when carrying out such washing of the lignophenol derivative with water, dispersion in water is carried out after carrying out only crushing, then agitation is carried out for approximately 2 hours, then the mixture is left to stand for 24 hours, and then the next day, in a state in which the lignophenol derivative-containing solid matter has settled naturally, the supernatant is discharged, and then fresh water is re-added and agitation is carried out; these steps are repeated, whereupon generally it has taken approximately 10 days until lignophenol derivative from which the sulfuric acid has been sufficiently removed (it is judged that the sulfuric acid has been removed once the pH of the dispersion has become at least 5) is recovered, and moreover solid matter remains in the supernatant, and hence the recovery rate has not been sufficiently high. In contrast with this, in the method according to the present invention described above, dispersion in water is carried out after carrying out crushing and making the solid matter ultra-fine, and then the dispersion is filtered using a vacuum filtration apparatus and the liquid is removed, whereby a sulfuric acid-containing liquid component can be removed efficiently, and moreover the loss of solid matter can be minimized. Moreover, by repeating the treatment of again dispersing the obtained lignophenol derivative in water and carrying out vacuum filtration, residual sulfuric acid in the lignophenol derivative can be completely removed efficiently. In the present invention, upon subjecting to vacuum filtration the solution obtained by crushing and making ultra-fine and then dispersing in 200 L of water the 35 kg of belt-shaped lignophenol derivative-containing solid matter separated from the reaction liquid obtained by carrying out the acid treatment on the 22.5 kg of p-cresol-impregnated cryptomeria wood powder, and then repeating the steps of again dispersing the solid matter obtained in 200 L of water and carrying out vacuum filtration 5 to 7 times, the pH of the filtrate became at least 5 and hence it was determined that the sulfuric acid had been sufficiently removed; this process of deacidifying/washing the lignophenol derivative, which has required approximately 10 days conventionally, could be completed in approximately 1 day. Moreover, the amount of the lignophenol derivative ultimately obtained was 6.5 kg (in terms of dry matter), and hence a yield double that conventionally obtained could be obtained.

### Example 3

1 kg of a cryptomeria wood powder obtained by carrying out only degreasing and drying treatment in a conical ribbon mixer (RIBOCONE made by Okawara Mfg. Co., Ltd.) as in Example 1 was put into a Lödige mixer (FMK type made by the German company Lödige), and while being agitated, 4 L of an acetone solution having 0.5 kg of p-cresol dissolved therein was sprayed on, thus impregnating the p-cresol into the cryptomeria wood powder. As a result, the p-cresol impregnation and solvent drying steps took 1 hour in total, and hence the time taken could be greatly reduced compared with the more than 2 days taken for the method of Example 1 in which the cryptomeria wood powder was put into approximately 10 times the amount of the p-cresol solution. Furthermore, the p-cresol was dispersed and impregnated into the cryptomeria wood powder well by using the small amount of p-cresol solution, and upon treating the p-cresol-impregnated wood powder thus obtained using the same procedure as in Example 1, a lignophenol derivative was obtained with at least the same yield and quality as in Example 1.

### INDUSTRIAL APPLICABILITY

According to the present invention, a lignocellulosic material can be treated so as to separate and recover a lignophenol derivative and sugar efficiently. Moreover, according to another form of the present invention, solid matter obtained through solid-liquid separation carried out on a reaction liquid after acid treatment is subjected to crushing and treatment to make the solid matter ultra-fine, and is then dispersed in water, whereby the recovery of the lignophenol derivative and the removal by washing of residual acid can be carried out much more efficiently and in a much shorter time than with a conventional method. Furthermore, according to another form of the present invention, in a step of impregnating the phenol derivative into the lignocellulosic material, a method of spraying a solution of the phenol derivative in an amount of approximately 1 to 5 times relative to the lignocellulosic material while agitating the wood powder is adopted, whereby the amount used of an organic solvent can be reduced, and moreover the time taken for the impregnation step can be greatly reduced.

## Claims

1. A method of preparing a lignophenol derivative and an acid/sugar solution, comprising subjecting a reaction mixture of a lignocellulosic material, a phenol derivative and an acid to solid-liquid separation so as to separate into a solid-phase lignophenol derivative and a liquid-phase acid/sugar mixture, and then subjecting the separated lignophenol derivative to deacidification/washing.

2. The method according to claim 1, wherein the reaction mixture of the lignocellulosic material, the phenol derivative and the acid is obtained by adding the acid to the lignocellulosic material which has been impregnated with the phenol derivative, and carrying out reaction at 20 to 40°C.

3. The method according to claim 2, wherein the temperature in the reaction is held at a constant temperature.

4. The method according to claim 2 or 3, wherein concentrated sulfuric acid of concentration at least 65% is added as the acid.

5. The method according to any of claims 1 through 4, wherein the solid-liquid separation is carried out on the reaction mixture of the lignocellulosic material, the phenol derivative and the acid using a hole-less bottom discharge type centrifugal separator.

6. A method of recovering a lignophenol derivative, comprising adding water to a lignophenol derivative obtained as a solid phase through solid-liquid separation carried out on a reaction mixture of a lignocellulosic material, a phenol derivative and an acid, and crushing so as to obtain a fine slurry, next dispersing the fine slurry obtained in water, and then recovering solid matter.

7. The method according to claim 6, wherein after the fine slurry has been dispersed in the water, the dispersion is subjected to second solid-liquid separation treatment using a filtration apparatus so as to recover the solid matter.

8. The method according to claim 6 or 7, further comprising steps of subjecting the recovered solid matter to rough drying at a temperature of not more than 60°C, and then to high-level drying using a vacuum microwave drying apparatus.

9. The method according to claim 8, wherein the recovered solid matter is dried to a water content of not more than 50% through the rough drying, and then dried to a water content of not more than 10% through the high-level drying.

10. A method of impregnating a phenol derivative into a crushed lignocellulosic material, the method comprising spraying 1 to 5 L of a solution of the phenol derivative in an organic solvent on the crushed lignocellulosic material per 1 kg of the lignocellulosic material while agitating the lignocellulosic material.

11. An apparatus for recovering a lignophenol derivative, comprising: a crushing apparatus that receives solid matter obtained through solid-liquid separation carried out on a reaction mixture of a lignocellulosic material, a phenol derivative and an acid, and is for crushing the solid matter; an agitating tank for adding water to the crushed solid matter and agitating; and a solid-liquid separation apparatus that receives an aqueous slurry recovered from the agitating tank, and is for carrying out solid-liquid separation.

12. An apparatus for recovering a lignophenol derivative, comprising: a first solid-liquid separation apparatus for carrying out solid-liquid separation on a reaction mixture of a lignocellulosic material, a phenol derivative and an acid; a crushing apparatus that receives solid matter recovered through the first solid-liquid separation, and is for crushing the solid matter; an agitating tank for adding water to the crushed solid matter and agitating; and a second solid-liquid separation apparatus that receives an aqueous slurry recovered from the agitating tank, and is for carrying out solid-liquid separation.

13. The apparatus according to claim 12, wherein the first solid-liquid separation apparatus is a hole-less bottom discharge type centrifugal separator.

14. An apparatus for recovering a lignophenol derivative, comprising: an acid treatment tank that receives a phenol derivative-impregnated lignocellulosic material, and is for adding an acid to bring about reaction; a first solid-liquid separation apparatus that receives a reaction mixture of the lignocellulosic material, the phenol derivative and the acid recovered from the acid treatment tank, and is for carrying out solid-liquid separation; a crushing apparatus that receives solid matter recovered through the first solid-liquid separation, and is for crushing the solid matter; an agitating tank for adding water to the crushed solid matter and agitating; and a second solid-liquid separation apparatus that receives an aqueous slurry recovered from the agitating tank, and is for carrying out solid-liquid separation.

15. The apparatus according to claim 14, wherein the acid treatment tank has means for holding the temperature constant during the reaction.

16. The apparatus according to claim 14 or 15, wherein the first solid-liquid separation apparatus is a hole-less bottom discharge type centrifugal separator.

17. An acid treatment reaction apparatus for reacting an acid with a phenol derivative-impregnated lignocellulosic material so as to produce a lignophenol derivative and an acid/sugar solution, the apparatus comprising: a reaction tank that receives the phenol derivative-impregnated lignocellulosic material and the acid, and is for carrying out the reaction; a warm water jacket provided on the outside of the reaction tank; means for supplying and discharging warm water into and out of the warm water jacket; a temperature measuring apparatus for measuring the temperature of the contents of the reaction tank; and control means for adjusting the temperature and flow rate of the warm water supplied into the warm water jacket in accordance with the temperature of the contents measured by the temperature measuring apparatus.
